## Europäisches Patentamt
## European Patent Office
## Office européen des brevets

(19)

(11) Publication number: **0 018 817**
A1

(12) # EUROPEAN PATENT APPLICATION

(21) Application number: **80301406.7**

(22) Date of filing: **29.04.80**

(51) Int. Cl.³: **A 61 M 5/16**

(30) Priority: **30.04.79 GB 7914959**

(43) Date of publication of application:
**12.11.80 Bulletin 80/23**

(84) Designated Contracting States:
**BE DE FR GB IT NL**

(71) Applicant: **Lale, Peter Gilbert**
**6, Manland Avenue**
**Harpenden Herts. AL5 4RF(GB)**

(72) Inventor: **Lale, Peter Gilbert**
**6, Manland Avenue**
**Harpenden Herts. AL5 4RF(GB)**

(74) Representative: **Smith, Martin Stanley**
**Stevens, Hewlett & Perkins 5, Quality Court Chancery Lane**
**London WC2A 1HZ(GB)**

(54) Method and apparatus for measuring drip rate.

(57) For measuring drip rate of fluid from the end (A) of a tube (T), particularly for medical infusion administration sets, a piezoelectric transducer arrangement (TD) is mounted against the tube (T) and transmits ultrasonic energy into the fluid. Signals resulting from reflections of the energy from the drops (DR) as they form and leave the tube (T) are processed electronically to give a drip rate indication.

FIG. 2

EP 0 018 817 A1

"METHOD AND APPARATUS FOR MEASURING DRIP RATE"

· The invention relates to a method and apparatus for measuring the drip rate of liquid which forms drops which drip from a tube. The invention is particularly useful in monitoring the rate of fluid infusion into medical patients.

The majority of administration sets for fluid infusion rely on gravity feed from a container placed high on a drip stand with the fluid passing down to a drip chamber, then down a plastic tube with a flow control and on into the patient. A nurse will normally set the drip rate at an estimate of the rate required, then count the rate against a watch, then readjust closer to the required rate and count again, and repeat this until the rate is correct. The rate is checked again from time to time, and if the tube has blocked it is cleared to start the flow once more. The blockage may have gone unnoticed for an appreciable time.

The present invention seeks to provide an improved method and apparatus for monitoring drip rate.

According to one aspect of the invention there is provided a method of measuring drip rate of fluid which forms drops which drip from a tube, the method consisting in transmitting ultrasonic waves into the fluid in the tube, receiving from the fluid in the tube ultrasonic waves reflected by the drops as they form, and processing signals transduced from the received waves to give an indication of drip rate.

- 2 -

According to another aspect of the invention, there is provided a drip-rate monitor for a fluid administration set, the monitor comprising an ultrasonic transducer acoustically coupled to a tube having an end from which the fluid drips in the form of drops, the transducer being capable of transmitting ultrasonic waves through the fluid in the tube and receiving ultrasonic waves reflected from the drops as they form and depart; and an electric circuit connected to the transducer for processing signals resulting from the received waves and giving an indication of drip rate.

The transducer is capable of being small and light and easily fitted in position. It does not obscure the nurse's view of the drip chamber, and further allows the chamber to be squeezed radiply when, for example, blood or blood derivatives have to be pumped rapidly into a patient in an emergency. If misting or droplet formation does occur inside the drip chamber, this can affect optical methods of observing drops, but has no effect on the proposed system. Moreover the drip rate may be observed by this means in many infusion sets without any modification of the sets.

The transducer described in the accompanying drawings is shown with separate transmission and receiving crystals. An alternative arrangement is to use a single crystal for both transmission and reception.

Preferably electronic circuits couple to the transducer for transmitting and receiving ultrasonic waves, processing the received signals, and providing pulses whose interval corresponds to the interval between drops. These pulses may be used to trigger further circuits to provide for example a digital display of drip rate and also alarm signals when the drip rate deviates too far from a predetermined setting.

The circuits in conjunction with the transducer provide ultrasonic waves whose wavelength is similar in magnitude to the dimensions of the drops. An upper limit to wavelength is set by the physical constraint of transmission down a tube of fixed diameter. The larger wavelengths result in smaller phase changes from unwanted disturbances, such as vibration causing surface movements at the drop surface or in the fluid surface above the tube in the filter chamber.

The circuits provide intermittent ultrasound pulses. By gating the power to the main current-carrying elements of the circuit, the circuit may be so arranged that the average power absorbed is low. The system may be battery operated, and a complete unit may be conveniently clamped to the drip stand with a short, light, connection to the transducer.

The invention will be further described with reference to the accompanying drawings of which :-

Figure 1 is a schematic side elevation of a conventional fluid administration set;

Figure 2 is an enlarged schematic side elevation of one embodiment of the invention showing the transducer in position above the drip chamber;

Figure 3 is a schematic plan view of Figure 2; and

Figure 4 is a circuit diagram of apparatus used in conjunction with the arrangement of Figures 1 and 2 for transmitting and receiving ultrasonic waves, with signal processing in order to obtain pulses whose interval corresponds to the interval between drops.

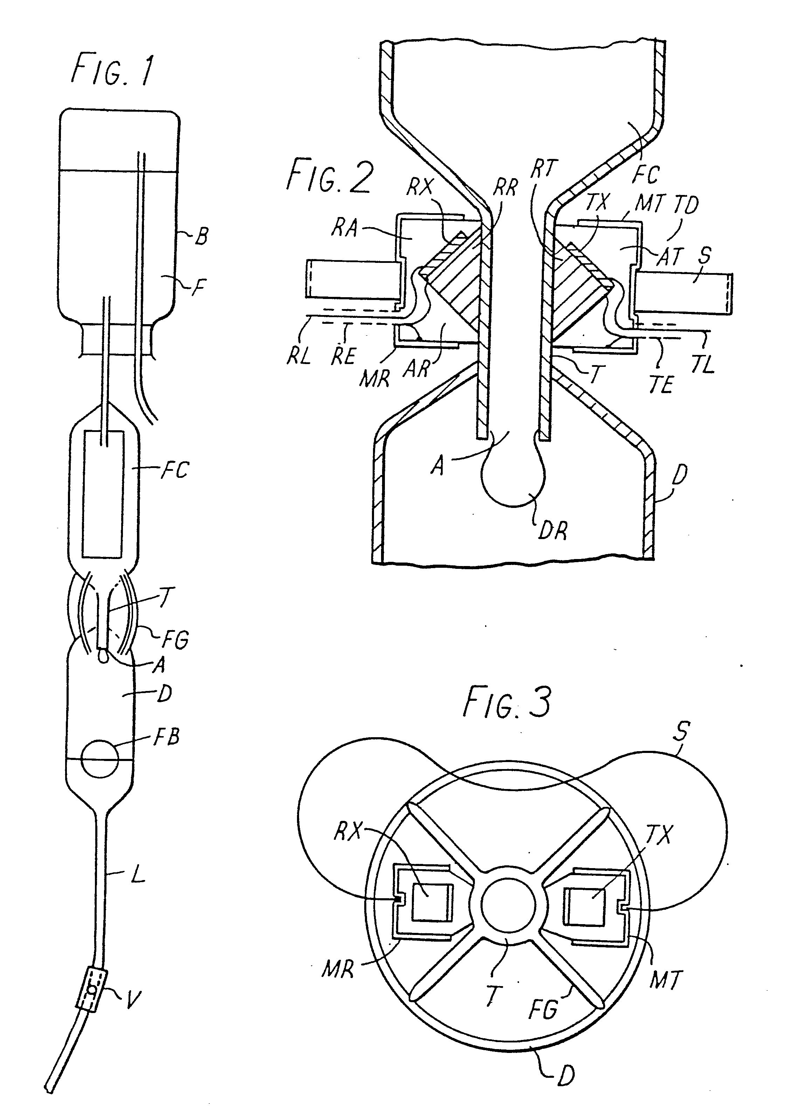

Referring to Figure 1 there is shown a fluid administration set comprising a reservoir bottle B for the infusion fluid F, a filter chamber FC which is normally kept full of fluid, through which the fluid passes to drip from aperture A in drip chamber D. A float ball FB floats on the surface of the fluid. Fluid flow is regulated by manual adjustment of a valve V in the outlet line L to the patient. The filter chamber FC is connected to the drip chamber D by means of a tube T. Support flanges FG are also present.

Figure 2 shows the present invention app lied. There is shown the base of the filter chamber FC, interconnecting tube T and the top of drip chamber D, with a transducer TD in position. The flanges (FG of Figure 1) are not shown in this view.

Transducer TD consists of two halves. In one half an ultrasonic crystal TX transmits ultrasonic waves through a rubber section RT into tube T. Some of the wave energy travels down to the drop DR and is then reflected back up tube T. Some of this wave energy enters the rubber section RR and reaches the ultrasonic receiver crystal RX.

In the transmitting half of the transducer, rubber section RT is shaped to fit closely against tube T. Crystal TX is bonded to a rubber section RT and the space between these and the metal housing MT is filled with absorbent rubber AT. Electrical connections TL and TE to the crystal are brought out in a screened lead, TE being connected to the metal housing.

Similarly in the receiving half of the transducer, rubber section RR is shaped to fit closely against tube T. Crystal RX is bonded to rubber section RR and the space between these and the metal housing MR is filled with absorbent rubber AR. Electrical connections RL and RE to the crystal are brought out in a screened lead, RE being connected to the metal housing.

The two transducer halves are held against tube T by means of a spring clip S.

Figure 3 shows a schematic plan view of Figure 2 with the filter chamber cut away. The flanges FG which may be present are indicated here. The transmitter crystal TX is shown in its metal housing MT and similarly the receiver crystal RX is shown in its metal housing MR. The spring clip S is indicated, holding the housings MT and MR against tube T.

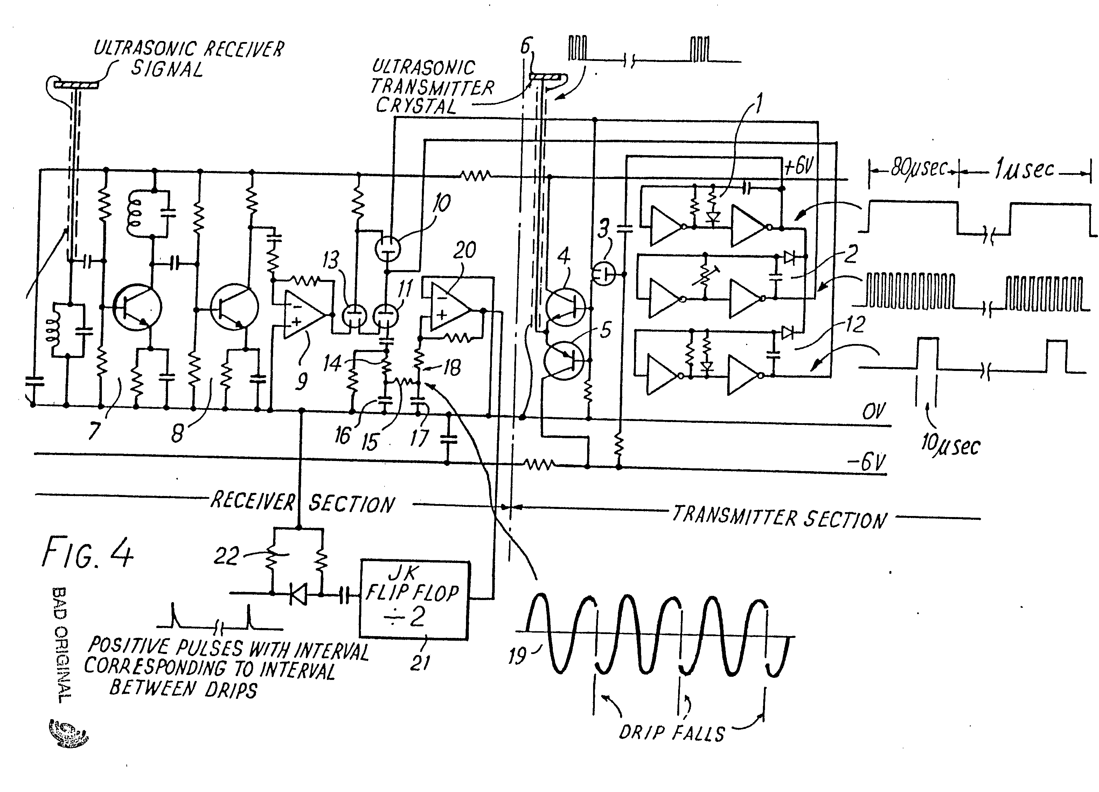

Referring to Figure 4 as astable multivibrator 1 provided 80$\mu$ sec positive pulses at intervals of 1 m.sec. These gate an astable multivibrator 2 which oscillates at the ultrasonic transmitter frequency. An analogue switch 3 is switched on for approximately 10μseconds each time multivibrator 1 goes positive and allows the output of multivibrator 2 to drive complementary transistors 4 and 5, which in turn drive the ultrasonic transmitter crystal 6.

Ultrasonic waves impinging on the receiver crystal create small sinusoidal voltages which are amplified by a tuned amplifier stage 7. A further stage of amplification 8 occurs before amplification by a wide-band operational amplifier 9. Analogue gates 10 and 11 are opened for 10μsecs by a delayed pulse derived from a gated astable multivibrator 12. The delay is set so that waves reflected from the drop surface are reaching the receiver crystal during the 10μsec. period. (Reflections from the fluid surface in the filter chamber normally occur at a later period, and therefore have no effect.) Phase sensitive rectification of the output of amplifier 9 occurs during this period as an analogue gate 13 is driven via a gate 10 from multivibrator 2. Resistors 14, 15 and capacitors 16, 17 remove high frequency components from the signal, so that the signal at 18 corresponds to the phase shifts that occur as each drop grows and then falls.

With the chosen ultrasonic transmitter frequency, during the growth of each drop four phase reversals occur. Thus two approximately sinudoidal waves occur at point 18 for each drip that is formed, with a fast transition as each drop falls. Waveform 19 indicates a typical output at point 18. The choice of transmitter frequency is governed by certain physical factors. If a high frequency is chosen, for example 2MHz or more, a well-defined beam of ultrasound is obtained. However, the wavelength in aqueous fluid is only 0.75 mm at 2MHz and therefore very small changes in the dimensions or position of the drop will cause phase reversal. This can occur through vibration or movement of the drip chamber. At lower frequencies, i.e. larger wavelengths, phase reversal requires larger linear changes and the output is less susceptible to interference. However, as the frequency is lowered, the wavelength increases to the point where it is larger than the diameter of the tube leading from the transmitter to the drip aperture. The proportion of ultrasonic energy reaching the drop and reflecting from it falls rapidly. In the transducer described, a frequency of approximately 330 kHz was chosen, as this gave four phase reversals with drips from a commonly used drip chamber, where 15 drips are equivalent to 1 ml. Apart from exceptionally dimensioned drip equipment, a frequency of operation 200 kHz and 500 kHz will generally be satisfactory.

A schmitt trigger 20 is used to square up the waveform 19, before feeding it to a divide-by-two counter 21. The output is then fed through the circuit 22 so that positive pulses are produced, whose interval corresponds to the interval between drips.

These pulses may then be used to trigger digital circuits, so that drip rate is displayed, for example by a liquid crystal display, and audible and or visual alarm circuits may be provided to indicate when the drip rate varies too far from its initial setting. A unit which houses the electronic circuits and display may be conveniently attached to the drip stand.

Since the formation of each drop is relatively slow, a slow repetition rate may be used for the ultrasonic pulses. Moreover the circuit may be so arranged that very little current is drawn until the onset of each ultrasonic pulse, by gating the power supplies to the operational amplifiers, and by the use of CMOS invertors and CMOS analogue gates. By these means it is possible to provide a small battery operated unit with low current drain.

The invention is not restricted to the particular embodiment described in the foregoing text and diagrams. The transducer may be used in any administration set where space is available above the drip chamber to make contact with the central tube. Those administration sets designed especially for very small drops (e.g. 60 drops per ml.) would require suitably selected ultrasound wavelengths.

## CLAIMS

1.      A method of measuring drip rate of fluid which forms drops which drip from a tube, the method consisting in transmitting ultrasonic waves into the fluid in the tube, receiving from the fluid in the tube ultrasonic waves reflected by the drops as they form, and processing signals transduced from the received waves to give an indication of drip rate.

2.      A drip rate monitor for a fluid administration set, the monitor comprising an ultrasonic transducer acoustically coupled to a tube having an end from which the fluid drips in the form of drops, the transducer being capable of transmitting ultrasonic waves through the fluid in the tube and receiving ultrasonic waves reflected from the drops as they form and depart; and an electric circuit connected to the transducer for processing signals resulting from the received waves and giving an indication of drip rate.

3.      A drip rate monitor as claimed in Claim 2 wherein the wavelength of the ultrasonic waves in the fluid is substantially the same as the dimensions of the drops which form.

4.      A monitor as claimed in Claim 2 wherein the circuit is effective to provide the ultrasonic waves in the form of intermittent pulses.

5.      A monitor as claimed in Claim 4 wherein the circuit is effective to gate the signals from the transducer so that received signals are applied from

- 10 -

the transducer to the circuit only at a particular period after an ultrasonic pulse is transmitted by the transducer into the fluid, so that spurious echo signals are rejected.

6. A monitor as claimed in Claim 3 wherein the frequency of the ultrasonic waves is between 200 kHz and 500 kHz.

7. A monitor as claimed in Claim 6 wherein the frequency of the ultrasonic waves is substantially 330 kHz.

FIG.1

B

F

FC

T

FG

A

D

FB

L

V

FIG.2

RX  RR  RT

RA  FC

TX  MT  TD

AT  S

RL  RE  MR  AR

T

TE  TL

A

DR

D

FIG.3

S

RX  TX

MR  MT

T  FG

D

ULTRASONIC RECEIVER SIGNAL

ULTRASONIC TRANSMITTER CRYSTAL

+6V

80μsec — 1μsec

OV

−6V

10μsec

RECEIVER SECTION — TRANSMITTER SECTION

FIG. 4

JK FLIP FLOP ÷ 2

POSITIVE PULSES WITH INTERVAL CORRESPONDING TO INTERVAL BETWEEN DRIPS

DRIP FALLS

European Patent Office

**EUROPEAN SEARCH REPORT**

| | DOCUMENTS CONSIDERED TO BE RELEVANT | | CLASSIFICATION OF THE APPLICATION (Int. Cl. ³) |
|---|---|---|---|
| Category | Citation of document with indication, where appropriate, of relevant passages | Relevant to claim | |
| | FR - A - 2 281 602 (UNION CHIMIQUE CONTINENTALE U.C.C.)  * Figures 1,2; page 1, lines 27-33; page 3, lines 3-25 *  -- | 1,2,4 | A 61 M 5/16 |
| | FR - A - 1 395 838 (SFIM)  * Figures ; page 1, right-hand column, lines 14-27 *  -- | 1,2 | |
| A | FR - A - 2 281 601 (UNION CHIMIQUE CONTINENTALE U.C.C.) | 1 | TECHNICAL FIELDS SEARCHED (Int.Cl. ³) |
| A | FR - A - 2 273 264 (CLIN MIDY)  ---- | 1 | A 61 M  G 06 M  B 01 L |

CATEGORY OF CITED DOCUMENTS

X: particularly relevant

A: technological background

O: non-written disclosure

P: intermediate document

T: theory or principle underlying the invention

E: conflicting application

D: document cited in the application

L: citation for other reasons

&: member of the same patent family, corresponding document

☒ The present search report has been drawn up for all claims

| Place of search The Hague | Date of completion of the search 01-08-1980 | Examiner HERBELET |
|---|---|---|

EPO Form 1503.1  06.78